# EUROPÄISCHE PATENTSCHRIFT

(11) **EP 4 188 288 B1**
(45) Veröffentlichungstag und Bekanntmachung des Hinweises auf die Patenterteilung: **02.09.2026**
(21) Anmeldenummer: 21758617.1
(22) Anmeldetag: 27.07.2021
(51) Int. Cl.: A61F 5/01, A61F 5/02, B29C 33/38

(54) **VERFAHREN ZUR HERSTELLUNG EINES ORTHOPÄDISCHEN STÜTZELEMENTS**
METHOD FOR PRODUCING AN ORTHOPAEDIC SUPPORT ELEMENT
PROCÉDÉ DE FABRICATION D'UN ÉLÉMENT DE SUPPORT ORTHOPÉDIQUE

(30) Priorität: 28.07.2020 DE 102020119920
(43) Veröffentlichungstag der Anmeldung: 07.06.2023
(73) Patentinhaber: Hahlbrock, David, 51061 Köln (DE)
(72) Erfinder: Hahlbrock, David, 51061 Köln (DE)
(74) Vertreter: Kreuels, Justus
(86) Internationale Anmeldenummer: PCT/EP2021/071063
(87) Internationale Veröffentlichungsnummer: WO 2022/023376

(56) Entgegenhaltungen:
- US-A- 3 541 646
- US-A- 5 836 902
- US-A1- 2011 092 867
- US-A1- 2019 336 317
- US-B1- 8 771 212

## Beschreibung

Die Erfindung betrifft ein Verfahren zur Herstellung eines orthopädischen Stützelements. Solche orthopädischen Stützelemente werden üblicherweise auch als "Orthesen" bezeichnet. Solche orthopädischen Stützelemente werden zur Korrektur von Fehlstellungen von Körperteilen oder zur Unterstützung bewegungseingeschränkter Körperteile verwendet, beispielsweise für Hand- oder Fußgelenke, die eine Fehlstellung aufweisen. Solche orthopädischen Stützelemente können an das Körperteil mit der Fehlstellung angelegt werden und üben einen sanften Druck auf das Körperteil aus. So wird die Fehlstellung korrigiert und/oder das Körperteil wird unterstützt.

Verschiedene Arten von orthopädischen Stützelemente sowie Verfahren zu deren Herstellung sind aus den Schriften US 5 386 902 A, US 8 771 212 B1 und US 2019/336317 A1 bekannt. Diese Dokumente offenbaren Stützelemente aufweisend Überlappungsabschnitte in welchen die Stützelemente mit sich überlappen, wenn sie an dem zu korrigierenden Körperteil anliegen.

Aus der US 2011/0092867 A1 ist es bekannt verschiedene flexible Strukturen für die Herstellung von Orthesen vorzubereiten, so dass dann jeweils die passendste flexible Struktur als Ausgangsmaterial für die Herstellung einer individuellen Orthese verwendet werden kann. Es wird auch ein Spritzgussverfahren beschrieben zur Herstellung der Orthese, wobei die Orthese einen Überlappungsabschnitt aufweist, in welchem die Orthese mit sich selbst überlappt, wenn sie an einem zu korrigierenden Körperteil angelegt ist.

Aus der US 3 541 646 A ist ein Spritzgussverfahren zur Herstellung eines Skischuhs bekannt.

Es ist bekannt, für die Herstellung solcher orthopädischen Stützelemente einen sogenannten Leisten als Ausgangsbasis zu verwenden. Der vorzugsweise aus Gips bestehende Leisten repräsentiert die äußere Form des zu korrigierenden Körperteils in korrigierter Stellung bzw. in unterstützter Stellung. Anhand dieses Leistens wird das orthopädische Stützelement erstellt. Außerdem ist es bekannt als Material für solche orthopädischen Stützelemente Silikon zu verwenden.

Gemäß einem bekannten Herstellungsverfahren für derartige orthopädische Stützelemente wird das Silikon mit Hilfe einer Walze auf die entsprechende Dicke gebracht und dann auf den Leisten aufgebracht. Dies geschieht häufig durch händisches Auftragen und In-Form-Bringen des Silikons auf dem Gipsleisten. Das orthopädische Stützelement, bzw. das Material des orthopädischen Stützelements wird an bestimmten Stellen (je nach therapeutischer Notwendigkeit) verstärkt. Gegebenenfalls werden später noch händisch Verschlussmechanismen und eine vom Gesetzgeber vorgeschriebene Kennzeichnung auf und an dem orthopädischen Stützelement befestigt.

Dieses Verfahren erlaubt allerdings nur beschränkte Kantenlängen des ausgewalzten Silikons und kann daher nicht für große Orthesen (z.B.Thorso genutzt werden). Dies liegt beispielsweise daran, dass nur Silikonwalzen mit bestimmten Kantenlängen verfügbar sind und daran, dass das Herstellungsverfahren nur bei orthopädischen Stützelementen bis zu einer bestimmten Größe genau durchführbar ist. Ein weiteres Problem dieses Verfahrens ist die Entlüftung des Materials (üblicherweise Silikon). Besonders aufwendig ist die Entgasung des Silikons, die händisch durch Aufstechen der Gasblasen auf der Walze erfolgt. Ein weiteres häufig auftretendes Problem ist das Absacken des Materials. Da das Silikon beim Auftragen noch nicht seine Endtopfzeit und Vulkanisationszeit erreicht hat, verläuft die Orthese und sackt sichtbar ab.

Ein weiteres bekanntes Herstellungsverfahren für derartige orthopädische Stützelemente/Orthesen ist das klassische Abformverfahren. Bei diesem Verfahren wird das Material des orthopädischen Stützelements in eine Gussform gebracht, die mit flüssigem Gußmaterial gefüllt wird. Dieses Gußmaterial kann beispielsweise auch Silikon sein. Allerdings ist die Herstellung einer Gussform für einen klassischen Abformprozess ähnlich aufwendig wie die Herstellung einer Silikonorthese im Walzverfahren. Hinzu kommen die hohen Anteile händischer Arbeit im Prozess des klassischen Abformverfahrens. Mit diesem Verfahren lassen sich allerdings keine orthopädischen Stützelemente mit einem Überlappungsabschnitt herstellen.

Hiervon ausgehend ist es Aufgabe der hier vorliegenden Erfindung die geschilderten Nachteile des Standes der Technik zu lösen bzw. zu umgehen oder zumindest zu lindern. Es soll insbesondere ein besonders vorteilhaftes Verfahren zur Herstellung eines orthopädischen Stützelementes gezeigt werden, welches die beschriebenen Nachteile der bekannten Verfahren lindert. Außerdem soll ein besonders vorteilhaftes, mit dem Verfahren herstellbares orthopädisches Stützelement angegeben werden, dass besonders einfach und präzise an das zu behandelnde Körperteil anlegbar ist.

Diese Aufgabe wird gelöst mit einem Verfahren gemäß den Merkmalen des Anspruchs 1 Weitere vorteilhafte Ausgestaltungen des Verfahrens sind in den abhängig formulierten Ansprüchen angegeben. Die in den abhängigen Ansprüchen und der Beschreibung angegebenen Ausführungsbeispiele sind in beliebiger, technologisch sinnvoller Weise miteinander kombinierbar.

Die Gußform ist (gedanklich) in eine Innenform und Außenform unterteilbar. In vielen Ausführungsvarianten sind die Innenform und die Außenform tatsächlich verschiedene Formteile. Der Innenform ist der Teil der Gußform zuzurechnen, welcher dem aus bekannten Verfahren zur Herstellung von orthopädischen Stützelementen üblichen Leisten entspricht. Alle weiteren Abschnitte der Gußform sind der Außenform zuzurechnen. Der weiter oben beschriebene Formabschnitt, welcher den Abstand bewirkt ist der Außenform zuzurechnen. Wenn in den folgenden Abschnitten die Innenform und die Außenform erläutert werden, dann bezieht sich dies immer sowohl auch mehrteilige Gußformen mit voneinander trennbarer Innenform und Außenform als auch auf einteilige Gußformen, bei denen Innenform und Außenform miteinander verbunden sind.

Das orthopädische Stützelement kann auch als Orthese bezeichnet werden. Es dient zur Korrektur einer orthopädischen Fehlstellung eines Körperteils. Zur Korrektur einer solchen Fehlstellung kann das orthopädische Stützelement an das Körperteil angelegt werden. Das Verfahren ist besonders für die Herstellung von Orthesen zur Stützung von Fuß- und Handgelenken geeignet mit welchen eine Fehlstellung von Fuß- oder Handgelenk therapiert werden kann. Hier umfasste Körperteile sind demnach beispielsweise Fuß- oder Handgelenke aber auch sämtliche anderen Körperteile auf welche Orthesen angewendet werden können, insbesondere Arme, Beine oder Armabschnitte bzw. Beinabschnitte, etc.. Ein hier umfasstes Körperteil ist aber auch der Oberkörper, weil mit einer Orthese auch die Wirbelsäule therapiert werden kann.

Mit dem orthopädischen Stützelement können Fehlstellungen eines Körperteils korrigiert werden. Beispielsweise ist es möglich bei einer unnatürlichen Stellung von Hand- oder Fußgelenk durch das orthopädische Stützelement einen sanften aber permanenten Druck auf das Hand- oder Fußgelenk auszuüben. Durch diesen sanften aber stetigen Druck, wird das Hand- bzw. Fußgelenk sehr schonend in seiner Stellung korrigiert.

Auch im Fußbereich kann ein solches orthopädisches Stützelement, bzw. eine solche Orthese angewendet werden. Dies kann beispielsweise geschehen, um das Gehen und Laufen zu unterstützen. Bei starken Deformationen des Fußes kann eine Form hergestellt werden, die es ermöglicht den deformierten Fuß auf dem Boden abzurollen.

Mit dem beschriebenen Verfahren sind sehr viel komplexer gestaltete orthopädische Stützelemente herstellbar als mit klassischen Verfahren zur Herstellung von orthopädischen Stützelemente (bzw. Orthesen), insbesondere weil die Freiheit der Gestaltung der Gußform sehr viel größer ist, als die Freiheit bei klassischen Herstellungsverfahren, die im wesentlichen darauf basieren ein lappenförmiges Ausgangsprodukt in die gewünschte Form des orthopädischen Stützelementes zu bringen.

Durch den beschriebenen Überlappungsbereich kann das orthopädische Stützelement nach Art eines Umschlags oder nach Art eines Wickels an das Körperteil angelegt werden. Der Überlappungsbereich ermöglicht insbesondere, dass das orthopädische Stützelement das Körperteil im fraglichen Bereich, in dem das orthopädische Stützelement wirken soll, das Körperteil vollständig abdeckt und kein freier Bereich verbleibt in welchem das Körperteil von dem orthopädischen Stützelement nicht abgedeckt bzw. überspannt ist. Bei orthopädischen Stützelementen ohne einen solchen Überlappungsbereich verbleibt immer ein Spalt, an welchem das Körperteil nicht von dem orthopädischen Stützelement abgedeckt ist. Ein solcher Spalt kann zwar eine sehr geringe bis zu Null gehende Breite haben. Trotzdem wird ein solcher Spalt von dem Träger eines orthopädischen Stützelementes regelmäßig als unangenehm empfunden.

Die in Schritt a) bereit gestellte Innenform wird üblicherweise auch als Leisten bezeichnet. Bei dem beschriebenen Verfahren bildet sie einen in die Gussform eingelegten Kern entsprechen. Die bereitgestellte Innenform repräsentiert bereits eine korrigierte Stellung, in welche das zu korrigierende Körperteil durch das orthopädische Stützelement gebracht werden soll. Diese Innenform kann beispielsweise durch einen Abdruck des zu korrigierenden Körperteils hergestellt werden, wobei die Form dieses Abdrucks anschließend im Sinne der auf das Körperteil anzuwendenden Therapie angepasst wurde. Wenn das Körperteil bspw. eine ungewünschte Schiefstellung hat, so wird der Abdruck für die Herstellung der Innenform so korrigiert, dass dieser Schiefstellung entgegengewirkt wird. Für die Herstellung der Innenform aus einem Abdruck existieren umfangreiche Verfahren, die von Orthopädietechnikern angewendet werden. Die Wahl eines geeigneten Verfahrens ist jedoch für die Durchführung des hier beschriebenen erfindungsgemäßen Verfahrens nicht entscheidend. Das hier beschriebene Verfahren kann mit Innenformen angewendet werden, die auf jede beliebige Art hergestellt wurden.

Die Innenform bzw. der Leisten basiert üblicherweise auf Messungen bzw. Erfahrungen und repräsentiert die therapeutischen Entscheidungen des Orthopädietechnikers. Die Erstellung der Innenform kann verschieden auf verschiedene Arten ausgeführt werden. Die Innenform /der Leisten repräsentiert alle therapeutischen Entscheidungen und bildet eine Art Grundlage für das hier beschriebene Verfahren.

Als spezielle Vorgehensweise zur Herstellung der Innenform soll hier auf digitale Methoden zur Herstellung der Innenform hingewiesen werden. Bei einer digitalen Methode zur Erstellung der Innenform erstellt der Orthopädietechniker ein digitales Modell (digitaler Leisten) der Messdaten und lässt in dieses Modell seine therapeutischen Entscheidungen einfließen. Die Innenform liegt dann als digitales Modell in einer Datei vor. Diese Datei wird mit einer CNC-Maschine subtraktiv oder additiv als Innenform hergestellt. Bevorzugt ist eine Herstellung im additiven FDM-3D Druck [FDM = Fused Deposition Modeling].

Gemäß einer kombiniert händisch/digitalen Methode zur Erstellung der Innenform erarbeitet der Orthopädietechniker einen klassischen Gipsleisten, also eine Innenform aus Gips, die anschließend dreidimensional gescannt und mit einer CNC-Maschine subtraktiv oder additiv hergestellt wird. Bevorzugt ist eine Herstellung im additiven FDM-3D Druck.

Gemäß einer rein händischen Methode zur Erstellung der Innenform bzw. des Leistens erarbeitet der Orthopädietechniker einen klassischen Gipsleisten, der erst später zur Erstellung der Außenform gescannt wird. Im eigentlichen Gussprozess Schritt c) wird der vom Orthopädietechniker händisch erstellte Leisten selbst als Innenform genutzt.

Anschließend an Schritt a) wird in Schritt b) eine zu der Innenform passende Außenform bereitgestellt. Die Innenform kann in die Außenform eingelegt werden, um die (vollständige) Gussform zur Herstellung des orthopädischen Stützelements zu bilden. Die Außenform kann beispielsweise anhand der Innenform erstellt werden, in dem auf die Innenform vollumfänglich ein Aufmaß aufgegeben wird, welches die spätere Dicke des durch das Verfahren zu erstellenden orthopädischen Stützelementes vorgibt.

Das orthopädische Stützelement wird bei der Erstellung der Außenform auf Basis der dreidimensionalen/digitalen Repräsentation der Innenform digital um die Innenform herum modelliert. Das digitale Verfahren erlaubt einen präzisen Überlappungsabschnitt auf Basis der Innenform herzustellen, welche mit klassischen, rein händischen Verfahren zur Erstellung von orthopädischen Stützelementen nicht herstellbar sind. Das orthopädische Stützelement ist damit präzise an den Patientenkörper angepasst und kann gleichzeitig einen derartigen Überlappungsabschnitt aufweisen.

In einer zweidimensionalen Aufsicht betrachtet wird das orthopädische Stützelement spiralförmig um die Innenform modelliert, so dass zwischen Außenform und Innenform ein ausgießbarer Hohlraum in Form eines offenen (spiralförmigen) Zylinders entsteht , dessen offenen Enden sich überlagern/überlappen. Dies geschieht durch eine geeignete Formung der Außenform, bei der die Außenform den Überlappungsabschnitt zumindest abschnittsweise beidseitig umgibt.

Die Gußform weist einen der Außenform zugehörigen Formabschnitt auf, welcher an der Innenform anliegt und welcher die offenen Enden (erstes offenes Ende und zweites offenes Ende des orthopädischen Stützelementes) für den Gießvorgang voneinander trennt. Dieser Formabschnitt stellt einen Abstand zwischen den offenen Enden bereit.

Die Außenform und die Innenform umschließen zusammengesetzt den ausgießbaren Hohlraum.

Entscheidend für die Herstellung eines orthopädischen Stützelementes mit dem beschriebenen Überlappungsbereich ist es, dass die Außenform (bzw. ein der Außenform zurechenbarer Formabschnitt der Gußform) den Überlappungsbereich zumindest abschnittsweise beidseitig umgibt. Hiermit ist gemeint, dass auf beiden Seiten des Bereichs des orthopädischen Stützelementes welcher zum Überlappungsbereich zählt während des Gußprozesses Material der Außenform Außenform (bzw. Material eines der Außenform zurechenbaren Formabschnittes der Gußform) existiert. Das orthopädische Stützelement ist üblicherweise flächig ausgeführt, so dass die beiden Seiten des orthopädischen Stützelementes klar identifizierbar sind. Mit dem Begriff "flächig" ist hier eine flächige Form gemeint, wenn das orthopädische Stützelement nicht am Körperteil anliegt, sondern von dem Körperteil abgelegt bzw. gewissermaßen "abgewickelt" ist. Das Material der Außenform befindet sich nun auf beiden Seiten. Die Außenform muss dabei nicht einteilig sein. Es ist sogar bevorzugt, dass die Außenform mehrteilig ist und jeweils unterschiedliche Teile der Außenform unterschiedliche Seiten des orthopädischen Stützelementes im Bereich des Überlappungsabschnitts abdecken.

Die Innenform/der Leisten wird üblicherweise spätestens zur Herstellung der Außenform mit einem 3D Scanverfahren digitalisiert. Auf Basis dieser 3D Daten wird eine mehrteilige Gussform mit CAD Software generiert und mit 3D-Drucktechnik hergestellt.

Um die Innenform und die Außenform zueinander ausrichten zu können, ist eine Justierung vorteilhaft. Die Justierung erfolgt durch mechanisches Verbinden der Formteile zueinander, z.B. durch ein Ineinanderklemmen der Formteile von Innen und Außenform z.B. mit Hilfe von Steck- oder Schraubverbindungen. Die Justierung kann beispielsweise durch Stifte an der Innenform und entsprechende Ausnehmungen bzw. Aussparungen an der Außenform gebildet sein, welche ineinander eingreifen.

Zum Scannen der Innenform ist es vorteilhaft, wenn an der Innenform Marker vorgesehen sind, welche mitgescannt werden, um später ein genaues Ausrichten /Positionierung von Leisten und Außenform zueinander zu erleichtern.

Solche zusätzlichen Marker vereinfacht die Modellierung und Justierung der Formteile von Innen- und Außenform zueinander. Solche zusätzlichen Marker sind vorzugsweise fest mit der Innenform verbunden und verbleiben auch während des Gussprozess an der Innenform. Dieses zusätzlichen Marker können an einer geeigneten Stelle an der Innenform befestigt sein, bevorzugt jedoch am proximalem (zum Körper hin ausgerichteten) Ende der Innenform, an dem (bei klassisch hergestellten Leisten) häufig auch ein Armierungseisen nicht von der Gipsform umschlossen ist. Vorzugsweise existiert mindestens ein solcher Marker an der Innenform. Besonders bevorzugt existieren so viele Marker oder ein derart ausgebildeter Marker, dass eine exakte dreidimensionale Positionierung und Orientierung der Innenform mit Hilfe der Marker möglich ist.

Wenn die Innenform mit Hilfe digitaler Methoden erzeugt wird, können die Justierungen und/oder Marker durch 3D-Druckverfahren direkt mit an der Innenform vorgesehen sein. Gleiches gilt natürlich für die Außenform. Bevorzugt liegen die zusätzlichen Strukturen (Justierungen und/oder Marker) nicht innerhalb des Hohlraums der in Schritt c) ausgegossen wird.

Das Konzept der Verwendung von Justierungen und Markern zum Scannen des Leistens in der richtigen Ausrichtung bzw. zum richtigen Positionieren von Leisten/Innenform und Außenform zueinander ist auch unabhängig von den weiteren hier beschriebenen Verfahrensschritten anwendbar.

Die Besonderheit der mit den Verfahrensschritten a) und b) bereitgestellten Gussform ist, dass diese Gussform einen Überlappungsabschnitt dadurch vorgibt, dass die Außenform (bzw. ein der Außenform zurechenbarer Formabschnitt der Gussform) den Überlappungsabschnitt beidseitig umgibt. Das orthopädische Stützelement ist flächig gebildet und hat damit zwei Seiten (im Folgenden auch als erste Seite und zweite Seite oder aber auch als Innenseite und Außenseite bezeichnet). Mit dem Begriff "Umgeben" ist in diesem Zusammenhang gemeint, dass die Außenform abschnittsweise sowohl die erste Seite als auch die zweite Seite bzw. sowohl die Innenseite als auch die Außenseite des orthopädischen Stützelementes vorgibt.

Durch das Zusammenfügen der einzelnen Teile der Außenform ist die Bereitstellung eines ausgießbaren Raums in Spiralform in der zweidimensionalen Aufsicht möglich, die den offenen dreidimensionalen Zylinder bildet der in Schritt c) ausgegossen werden kann.

In Schritt c) wird das orthopädische Stützelement durch das Einfüllen eines Gußwerkstoffs in einen Zwischenraum zwischen Innenform und Außenform bzw. in der Gußform erzeugt. Bis zum Zeitpunkt des Gusses gibt es im gesamten Prozess keine Positivform des orthopädischen Stützelementes. Die Formfindung für das Produkt findet digital statt. Dies stellt einen beachtlichen Vorteil gegenüber bekannten Verfahren zu Erstellung derartiger orthopädischer Stützelemente dar.

In Schritt c) kann jedes beliebige Gußverfahren angewendet werden, insbesondere auch Spritzguß, Druckguss oder Vakuumguss. Besonders bevorzugt ist ein Gußverfahren, welches in einem Vakuum oder zumindest einem Teilvakuum mit einem atmosphärischen Druck unter 0,5 bar ausgeführt wird, weil hierdurch die Bildung von Blasen beim Gußverfahren besonders effektiv vermieden werden kann.

Besonders vorteilhaft ist das Verfahren, wenn die Außenform im Bereich des Überlappungsabschnitts eine Oberflächenform aufweist, die zumindest abschnittsweise der Oberflächenform der Innenform entspricht.

Wenn das orthopädische Stützelement an das Körperteil angelegt ist, existieren im Überlappungsabschnitt vorzugsweise zwei übereinander liegende Zonen des orthopädischen Stützelementes. Diese übereinander liegenden Zonen entsprechen bevorzugt dem ersten offenen Ende und dem zweiten offenen Ende des orthopädischen Stützelementes. Dabei liegt eine innere Oberfläche des orthopädischen Stützelementes an dem zu therapierenden Körperteil an. In dem Überlappungsabschnitt liegt darüber hinaus ein Teil der inneren Oberfläche des orthopädischen Stützelementes an einer äußeren Oberfläche des orthopädischen Stützelementes an. Vorzugsweise geht der am Körperteil anliegende Bereich der inneren Oberfläche stufenlos in den an der äußeren Oberfläche des orthopädischen Stützelements anliegenden Bereich des orthopädischen Stützelementes über. Dazu ist es regelmäßig notwendig, dass eine Oberfläche der Außenform zumindest abschnittsweise (bereichsweise) der Oberfläche der Innenform entspricht, weil so ein sanfter, stufenloser Übergang zwischen dem im Überlappungsabschnitt an der äußeren Oberfläche des orthopädischen Stützelements anliegenden Bereich der inneren Oberfläche und dem am Körperteil anliegenden Bereich der inneren Oberfläche ermöglich ist. Das Areal in dem sich die Oberflächenformen von Innen und Außenform entsprechen, kann eine Fläche oder auch ein linienförmiger Bereich sein.

Weiterhin vorteilhaft ist das Verfahren, wenn die Außenform einen Übergangsbereich zu dem Überlappungsabschnitt bildet in welchem die Außenform flächig an der Innenform anliegt.

In diesem Bereich entspricht die Form der inneren Oberfläche des orthopädischen Stützelementes welches mit dem Gußverfahren hergestellt wurde auch nicht exakt der Form der Innenform bzw. des Leistens vielmehr existiert hier ein Abstand zwischen der Innenform bzw. der Form des Leistens und der inneren Oberfläche, welcher durch den Formabschnitt der Gußform vorgegeben ist, welcher den Überlappungsabschnitt vorgibt.

Dieser Abstand bedingt auch, dass das orthopädische Stützelement gegenüber einer durch eine Gußform vorgegebenen Form (geringfügig) gebogen ist, wenn es an dem zu therapierenden Körperteil angelegt ist. Diese Biegung ist allerdings so gering, dass die therapeutische Wirkung des orthopädischen Stützelementes hierdurch nicht beeinträchtigt wird. Der durch den Formabschnitt bzw. durch die Gußform erzeugte Abstand beträgt bevorzugt weniger als 10 mm [Millimeter], besonders bevorzugt weniger als 5 mm, aber üblicherweise mehr als 0,5 mm bevorzugt mehr als 1 mm, damit eine ausreichend stabile Trennung der offenen Enden des orthopädischen Stützelementes beim Gießvorgang oder bei jedem anderen beliebigen Verfahren zur Herstellung des orthopädischen Stützelementes erreicht wird. Dieser Abstand kann beim Anlegen des orthopädischen Stützelementes an ein zu therapierendes Körperteil durch eine sehr geringfügige Biegung des orthopädischen Stützelementes kompensiert werden, welche keine Auswirkung auf die therapeutische Wirkung des orthopädischen Stützelementes hat. Die Form, in der das orthopädische Stützelement hergestellt wird und in welcher zwischen den beiden offenen Enden des orthopädischen Stützelementes der Abstand bereitgestellt wird, wird hier als Herstellungsformung bezeichnet. Die Form, in welcher das orthopädische Stützelement an das zu therapierende Körperteil angelegt ist und gegenüber der Herstellungsformung (geringfügig) gebogen ist wird als Therapieformung bezeichnet.

Es ist also eine flächige Anlagefläche zwischen der Außenform und der Innenform im Überlappungsbereich gebildet. An dieser flächigen Anlagefläche besteht ein für den Gußwerkstoff dichter Kontakt zwischen der Innenform und der Außenform, welcher dazu führt, dass der Gußwerkstoff, welcher später das orthopädische Stützelement bildet in diesem Bereich nicht mehr an der Innenform anliegt, sondern beidseitig von der Außenform umgeben ist. Die Ausbildung des Überlappungsbereichs wird dadurch ermöglicht, dass die Außenform das orthopädische Stützelement abschnittsweise beidseitig umgibt.

Darüber hinaus voreilhaft ist das Verfahren, wenn die Außenform mehrteilig ausgeführt ist und ein erster Teil der Außenform eine erste Seite des Überlappungsabschnittes vorgibt, während ein zweiter Teil der Außenform eine der ersten Seite gegenüberliegende zweite Seite des Überlappungsabschnittes vorgibt.

Die Außenform besteht vorzugsweise aus mindestens zwei oder mehr Teilen. Um das gegossene orthopädische Stützelement später aus der Gussform lösen zu können, ist eine Unterteilung der Außenform vorteilhaft. Alternativ kann auch eine einteilige Außenform oder sogar eine einteilige Gussform, bei welchem Innenform und Außenform zusammen mit einem 3D-Drucker ausgedruckt werden im Sinne der Verfahrensschritte a) und b) hergestellt werden. Dann sind die Schritte a) und b) miteinander integriert. Bei solchen Ausführungsvarianten ist dann aber üblicherweise eine Zerstörung der Gussform (Innenform und/oder Außenform) notwendig, um das fertige orthopädische Stützelement aus der Gussform zu lösen. Dies kann beispielsweise durch chemische Auflösung der Gussform oder Wegbrennen der Gussform erfolgen.

Besonders bevorzugt ist sogar, wenn die Außenform dreiteilig ausgeführt ist. Dann umgeben ein erster Teil und ein zweiter Teil wie beschrieben den Überlappungsabschnitt beidseitig. Darüber hinaus existiert ein dritter Teil, welcher eine dem Überlappungsabschnitt gegenüberliegenden Teil der Außenform bildet. Der Begriff "gegenüberliegend" bezeichnet hier insbesondere einen Bereich einer gegenüberliegenden Seite der Innenform.

Außerdem vorteilhaft ist das Verfahren, wenn zumindest die Außenform mit einem dreidimensionalen Druckverfahren hergestellt wird.

Hier sind insbesondere additiven Druckverfahren erfasst. Alternativ ist es aber auch möglich dreidimensionale subtraktive Verfahren, wie z.B. CNC Fräsen zur Herstellung der Außenform zu verwenden.

Bei der Herstellung der Außenform können verschiedene Strukturen mit erzeugt werden, die später zum problemlosen Durchführen des Gusses in Schritt c) dienen. Beispielsweise können Angusskanäle, Speiser und Steiger vorgesehen werden.

Dreidimensionale Druckverfahren ermöglichen die Herstellung von Objektenaus verschiedenen Materialien die auf digitalen dreidimensionalen Modellen basieren. Das digitale Modell wird dabei additiv mit computergesteuerten Werkzeugmaschinen schichtweise aufgebaut. Es bestehen verschieden Verfahren, die unter dem Begriff 3D-Druck zusammengefasst werden. Beispielsweise selektives Lasersintern (SLS), Fused Deposit Modelling (FDM). Alle Möglichkeiten, die digitale 3D Modelle mit additiven Verfahren formen können für die Herstellung des orthopädischen Stützelements genutzt werden.

Die Verwendung eines dreidimensionalen Druckverfahrens vereinfacht die effiziente Herstellung einer derart komplexen Außenform, wie sie für die Ausbildung des beschriebenen Überlappungsabschnitts notwendig ist.

Auch vorteilhaft ist das Verfahren, wenn der Gußwerkstoff ein Silikonwerkstoff ist.

Silikone vertragen sich besonders gut mit dem Körper. Zum einen ist das Material ungiftig für Menschen und Tiere, zum anderen ist das Material angenehm auf der Haut zu tragen. Der therapeutische Vorteil von weichem Material wie Silikon liegt darin, dass die daraus gefertigte Orthese die noch bestehende Funktionalität des Körpers unterstützt. Anders als mit harten Materialien sind Bewegungen möglich und das behandelte Körperteil versteift nicht im Therapieverlauf.

Gemäß weiteren Ausgestaltungen können mit dem Verfahren auch Elemente an dem orthopädischen Stützelement produziert werden, deren Genauigkeit höher ist als die Genauigkeit, die durch das 3D-Druckverfahren zur Herstellung der Außenform erreicht werden kann. Dazu können vorgefertigte Objekte (bspw. Siegel oder Ähnliches) mit in die Außenform integriert werden. Die hohe Verarbeitungsgenauigkeit von Silikon als Gußwerkstoff erlaubt allerdings bereits feinste Auflösungen. Funktionale Elemente aus anderen Materialien beispielsweise Verschlüsse, Verstärkungen oder Adapter zu anderem orthopädischen Geräten können integriert und in der Form justiert werden und in softwareseitig generierte Geometrien eingegossen werden. Es ist auch möglich funktionale Strukturen aus verschiedenen Materialien mit einem 3d Drucker in die Außenform oder die Innenform zu integrieren, die beim Gießen in Schritt c) eingegossen werden. Nach dem Guss werden die Elemente von der Form gelöst und verbleiben in der Orthese. 3D Drucker können verschiedene Materialien mit verschiedenen Eigenschaften drucken und je nach 3D Druckverfahren auch die Eigenschaften eines Materials in einem Druckprozess verändern. Damit ist es möglich ist Form und funktionale Teile in einem Druckprozess herzustellen oder die verschiedenen Teile vorzuproduzieren und später zusammenzusetzen. Spezielle, mit einem flüssigen Lösungsmittel lösbare 3d Druckmaterialien ermöglichen Hohlräume im inneren der Orthese durch lösliche Kerne.

Außerdem können in die Außenform und die Innenform funktionale Geometrien des orthopädischen Stützelements integriert werden, wie beispielweise Verschlüsse, ein teilweise überlappender und sich verjüngender Rand, Versteifung durch stärkere Materialdicken , Elastizitätssteigerungselemente , Mittel zur Gewichtsreduktion, wie beispielsweise poröse Körper und/oder Belüftungsstrukturen. Insbesondere durch in die Außenform modellierte Aussparungen können Kennzeichnungen erfolgen. Hierzu zählen beispielsweise Patientendaten, Herstellungsdatum und Informationen zu der herstellenden Orthopädiewerkstatt. Weiter können individuelle, persönliche ästhetische Designs in das orthopädische Stützelement eingearbeitet werden.

In Schritt c) können auch mehrstufige Gußverfahren angewendet werden. In mehrstufigen Gussverfahren können Elemente aus anderen Materialien und/oder aus Silikon mit unterschiedlichen Härtegraden verarbeitet und integriert werden. Hierdurch lassen sich unterschiedliche Härten des orthopädischen Stützelementes vorsehen. Durch das Herauslösen geschlossener Hohlräume, die in weiteren Prozessen ausgegossen werden, ist es möglich verschiedene Gußmaterialien gezielt an bestimmten Positionen innerhalb des orthopädischen Stützelementes vorzusehen. Hierzu wird vorzugsweise eine Form aus einem nichtlöslichen Material hergestellt, die durch Unterformen oder Kerne aus lösbaren Materialien ergänzt wird. Dann wird zuerst das Silikon in den Hohlraum der Form gegossen. Nach der Vernetzung werden die Unterformen oder Kerne schrittweise herausgelöst. Die neu entstandenen Hohlräume der getrockneten Form werden ausgegossen.

Nach Abschluss des Gußprozesses (Schritt c)) wird das orthopädische Stützelement zunächst aus der Gussform (gebildet aus Innenform und Außenform) gelöst. Dazu können Trennmittel eingesetzt werden. Als Trennmittel eignet sich beispielsweise Teflonspray oder Wachs. Ggf. kann das Herauslösen aus der Gussform auch mit Druckluftunterstützung erfolgen.

Durch das beschriebene Verfahren lassen sich orthopädische Stützelemente herstellen, die handwerklich eine höhere Qualität aufweisen als mit dem bekannten Walzverfahren hergestellte orthopädische Stützelemente. Beispielsweise können Gasansammlungen an funktionalen Geometrien (zum Beispiel in der Nähe von Verstärkungen des orthopädischen Stützelements) effektiv vermieden werden. Darüber hinaus kann eine eindeutige, präzise Form des gesamten orthopädischen Stützelementes garantiert werden.

Darüber hinaus sind bestimmte, besonders komplexe Geometrien mit den klassischen Verfahren nicht oder nur mit massivem Zeiteinsatz herstellbar. Solche besonders komplexen Geometrien lassen sich mit dem beschriebenen Verfahren präzise herstellen. Außerdem wird der therapeutische Anwendungsbereich derartiger orthopädischer Stützelemente erweitert. Im Gegensatz zur klassischen Herstellungstechnik, die nur Hand und Fußorthesen ermöglicht, können mit dem hier beschriebenen Verfahren orthopädische Stützelemente für alle Körperbereiche in sämtlichen Größen aus Silikon hergestellt werden. Dies gilt beispielsweise auch für orthopädische Stützelemente für den gesamten Torso eines Menschen.

Hier auch beschrieben werden soll ein nicht beanspruchtes orthopädisches Stützelement zur Korrektur einer orthopädischen Fehlstellung eines Körperteils, welches an das zu korrigierende Körperteil angelegt werden kann, aufweisend einen Überlappungsabschnitt, in welchem das orthopädischen Stützelement mit sich selbst überlappt, wenn es an dem zu korrigierenden Körperteil angelegt ist.

Dieses orthopädische Stützelement ist (wie weiter oben beschrieben) mit dem beschriebenen Gußverfahren hergestellt.

Die für das beschriebene Verfahren erläuterten besonderen Vorteile und Ausgestaltungsmerkmale sind in analoger Weise auf das orthopädische Stützelement anwendbar und übertragbar. Gleiches gilt für die im Folgenden beschriebenen besonderen Vorteile und Ausgestaltungsmerkmale des orthopädischen Stützelements, die in beliebiger Weise auf das Verfahren anwendbar und übertragbar sind.

Das orthopädische Stützelement ist gegenüber einer Fertigungsformung (die es während der Herstellung hatte) geringfügig verbogen und in eine Therapieformung gebracht, wenn es an dem zu korrigierenden Körperteil angelegt ist.

Die Herstellungsformung und die Therapieformung unterscheiden sich dadurch, dass in der Herstellungsformung ein Abstand zwischen den beiden offenen Enden des orthopädischen Stützelementes vorliegt und diese beiden offenen Enden in der Therapieformung aneinander anliegen. In der Herstellungsformung ist das Material des orthopädischen Stützelementes bevorzugt vollkommen entspannt. In der Therapieformung existiert eine (sehr geringfügige) Biegung, um welche das orthopädische Stützelement derart verbogen ist, dass die beiden offenen Enden (im Überlappungsabschnitt) aneinander anliegen. Der Abstand der beiden offenen Enden in der Herstellungsformung beträgt bevorzugt weniger als 10 mm [Millimeter], besonders bevorzugt weniger als 5 mm, aber üblicherweise mehr als 0,5 mm oder sogar mehr als 1 mm damit eine ausreichend stabile Trennung der offenen Enden des orthopädischen Stützelementes beim Gießvorgang erreicht wird.

Weiter bevorzugt ist das orthopädisches Stützelement bestehend aus Silikon.

Außerdem bevorzugt ist, wenn das orthopädische Stützelement mindestens eines der folgenden Strukturmerkmale aufweist, die mit dem Gußverfahren zur Herstellung des orthopädischen Stützelemente mit hergestellt wurden:
- mindestens ein Verschlussmittel,
- mindestens eine Einlage,
- mindestens eine Verzierung oder
- mindestens eine Kennzeichnung.

Solche Strukturmerkmale können insbesondere auch dadurch erzeugt werden, dass vorgefertigte Objekte mit in die Gußform integriert werden, die dann die genannten Strukturmerkmale zumindest teilweise bilden. Alternativ oder zusätzlich können solche Strukturmerkmale beim Gießprozess aus dem Material des orthopädischen Stützelementes mit gefertigt werden. Sämtliche weiter oben im Rahmen des beschriebenen Verfahrens offenbarten Details können zur Ausbildung von (zusätzlichen) Strukturmerkmalen an dem orthopädischen Stützelement beitragen.

Die Erfindung sowie das technische Umfeld werden nachfolgend anhand der Figuren näher erläutert. Die Figuren verdeutlichen besonders Ausführungsvarianten, auf die die Erfindung jedoch nicht begrenzt ist. Insbesondere ist darauf hinzuweisen, dass die Figuren und die darin dargestellten Größenverhältnisse nur schematisch sind. Es zeigen:
Fig. 1: Eine erste Schnittansicht durch eine Gussform zur Herstellung eines orthopädischen Stützelements,
Fig. 2: Eine zweite Schnittansicht durch eine Gussform zur Herstellung eines orthopädischen Stützelements,
Fig. 3: Eine Schnittansicht durch eine weitere Gussform zur Herstellung eines orthopädischen Stützelements,
Fig. 4: Eine Schnittansicht durch noch eine weitere Gussform zur Herstellung eines orthopädischen Stützelements,
Fig. 5: eine Schnittansicht durch eine weitere Gussform zur Herstellung eines orthopädischen Stützelements, und
Fig. 6: ein orthopädisches Stützelement in der Herstellungsformung
Fig. 7 das orthopädische Stützelement aus Fig. 6 in der Therapieformung.

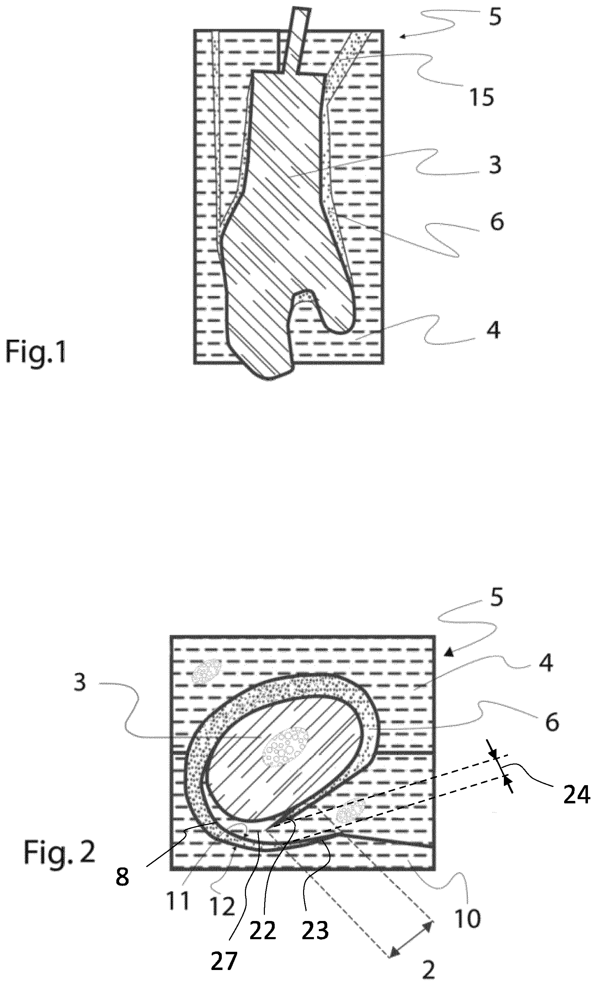

Fig. 1 zeigt Schnittansichten durch eine Gussform 5 für die Herstellung eines beschriebenen orthopädischen Stützelements, wobei diese Gussform aus Innenform 3 und Außenform 4 gebildet ist. Die Außenform weist dabei auch Angußkanäle 15 auf, die den Gießprozess zur Herstellung des orthopädischen Stützelementes unterstützten. In der Gussform 5 ist gemäß Fig. 1 der Gußwerkstoff 6 dargestellt aus welchem das orthopädische Stützelement hergestellt ist. Durch diese Angußkanäle 15 kann einerseits der Gußwerkstoff 6 zur Herstellung des orthopädischen Stützelements in die Gussform 5 eintreten. Andererseits kann durch derartige Kanäle Gas aus der Gussform 5 austreten, wenn der Gußwerkstoff 6 in die Gussform 5 eintritt.

Fig. 2, 3 und 4 zeigen einen schematischen Querschnitt von Gussformen 5 gemäß Fig. 1. Hier auch dargestellt ist der Überlappungssabschnitt 2. Der Überlappungsabschnitt 2 wird von einem der Außenform 4 vorgegebenen Formabschnitt 27 gebildet. An den Überlappungsabschnitt 2 schließt sich der Übergangsbereich 8 an, in welchem ein Übergang von der Innenform 3 zur Außenform 4 gebildet. In dem Übergangsbereich 8 liegt der Formabschnitt 27 an der Innenform 3 an. Durch den Formabschnitt 27 werden ein erstes offenes Ende 22 und ein zweites offenes Ende 23 des orthopädischen Stützelementes bzw. der Gußform 5 mit einem Abstand 24 zueinander vorgegeben. Die in Fig. 2 dargestellte Gußform ist dreiteilig ausgeführt mit einer Innenform 3 und einer Außenform 4, die wiederum aus einem ersten Teil 9 und einem zweiten Teil 10 besteht. Die Gußform 5 gibt eine erste (innere) Seite 11 und eine zweite (äußere) Seite 12 des orthopädischen Stützelementes vor. Diese beiden Seiten liegen sich im Bereich des Überlappungsabschnittes 2 gegenüber. Zwischen diesen beiden Seiten befindet sich der Formabschnitt 27.

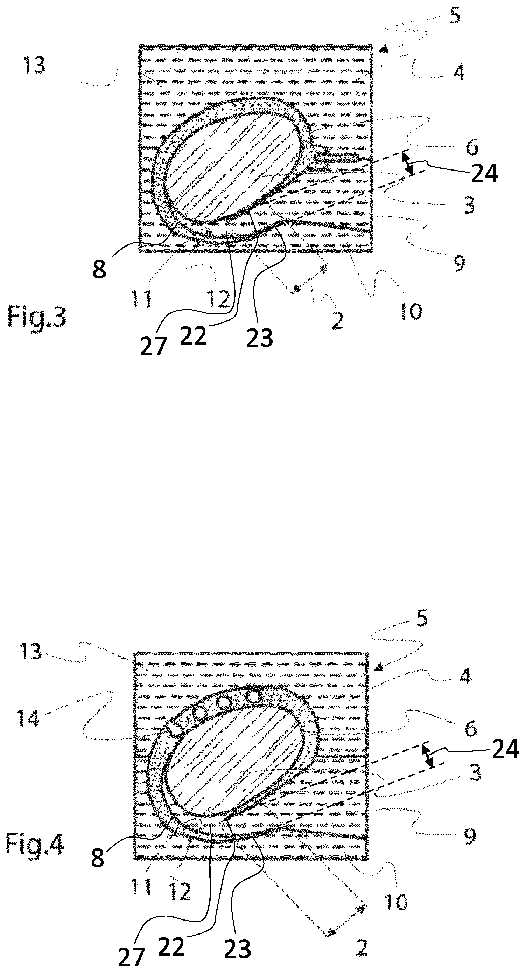

Fig. 3 und Fig.4 zeigen Schnittansichten durch zwei weitere verschiedene Gußformen 5 für das beschriebene Verfahren. Zu erkennen sind auch hier jeweils die Innenform 3 und die Außenform 4. Die Außenform besteht hier jeweils aus mehreren Teilen, nämlich einem ersten Teil 9, einem zweiten Teil 10 und einem dritten Teil 13. Die Außenform 4 und die Innenform 3 bilden zusammen einen zylinderförmigen (spiraligen) hohlen Bereich, in welchen der Gußwerkstoff 6 eingefüllt wird, um das orthopädische Stützelement herzustellen. Von der Außenform 4 wird ein Überlappungsabschnitt 2 des orthopädischen Stützelementes ausgebildet. In diesem Überlappungsabschnitt überlappt sich das orthopädische Stützelement. Ein erster Teil 9 der Außenform 4 bestimmt dabei die Form einer ersten Seite 11 des Überlappungsbereichs 2. Ein zweiter Teil 10 der Außenform 4 bestimmt die Form einer zweiten Seite 12 des Überlappungsbereichs 2. Der dritte Teil 13 ist auf der dem ersten Teil 9 und dem zweiten Teil 10 gegenüberliegenden Seite der Innenform 3 angeordnet. Der erste Teil 9 und der zweite Teil 10 bilden zusammen eine Halbschale der Gussform 5, während der dritte Teil 13 die andere Seite des Gussform 5 bildet.

Fig. 4 zeigt als Besonderheit beispielhaft, dass Einlagen 14 in das die Gussform 5 eingelegt sind, die von dem Gußwerkstoff 6 beim Gießprozess in Schritt c) ummantelt werden.

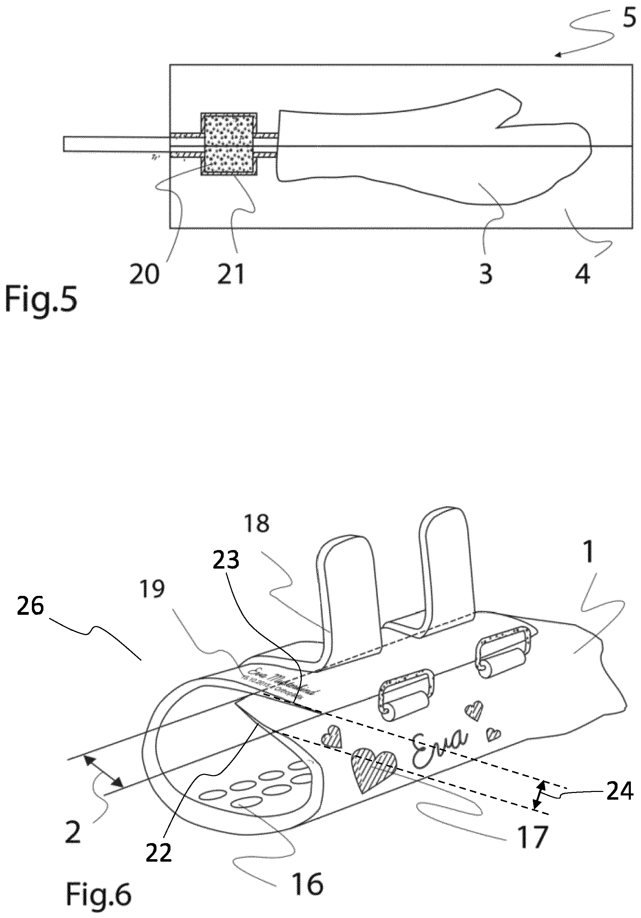

Fig. 5 zeigt eine Justierung mit der Innenform 3 und Außenform 4 relativ zueinander arretiert werden. Beispielsweise ist hier eine Ausnehmung 21 in der Außenform 4 und ein Stift 20 an der Innenform 3 gezeigt.

Fig. 6 zeigt ein fertiges orthopädisches Stützelement 1, welches nach dem beschriebenen Verfahren hergestellt wurde in einer Fertigungsformung 25, die insbesondere der durch die Gußform vorgegebenen Form entspricht. Durch das Herstellungsverfahren können an dem orthopädischen Stützelement problemlos Belüftungen 16, Verzierungen 16, Verschlußelemente 18 oder sonstige Anbauteile sowie Individualsierungen und Kennzeichnungen 19 vorgesehen werden. In Fig. 6 dreidimensional zu erkennen ist der Überlappungsabschnitt 2, in welchem sich ein erstes offenes Ende 22 und ein zweites offenes Ende 23 des orthopädischen Stützelementes 1 überlappen. In der Fertigungsformung existiert zwischen dem ersten offenen Ende 22 und dem zweiten offenen Ende 23 ein Abstand 24.

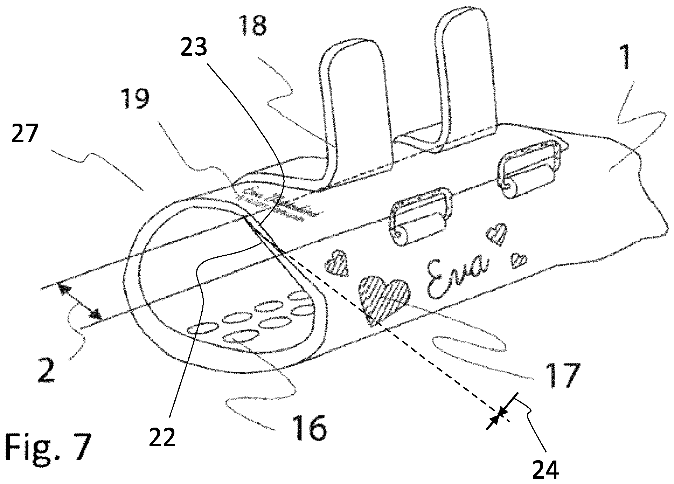

Fig. 7 zweigt das orthopädische Stützelemente 1 gemäß Fig. 6 in einer Therapieformung 26, wobei das zu therapierende Körperteil hier jedoch nicht dargestellt ist. Die Therapieformung 26 und die Fertigungsformung 25 unterscheiden sich dadurch, dass das erste offene Ende 22 und das zweite offene Ende 23 in der Therapieformung 26 aneinander anliegen demnach kein Abstand 24 vorliegt.

### Bezugszeichenliste

- 1.: orthopädisches Stützelement
- 2.: Überlappungsabschnitt
- 3.: Innenform
- 4.: Außenform
- 5.: Gussform
- 6.: Gußwerkstoff
- 7.: Oberflächenform
- 8.: Übergangsbereich
- 9.: Erster Teil
- 10.: Zweiter Teil
- 11.: Erste Seite
- 12.: Zweite Seite
- 13.: Drittes Teil
- 14.: Einlage
- 15.: Angußkanal
- 16.: Belüftung
- 17.: Verzierung
- 18.: Verschlusselement
- 19.: Individualisierung
- 20.: Stift
- 21.: Ausnehmung
- 22.: erstes offenes Ende
- 23.: zweites offenes Ende
- 24.: Abstand
- 25.: Fertigungsformung
- 26.: Therapieformung
- 27.: Formabschnitt

## Patentansprüche

1. Verfahren zur Herstellung eines orthopädischen Stützelements (1), aufweisend einen Überlappungsabschnitt (2), in welchem das orthopädischen Stützelement mit sich selbst überlappt, wenn es an einem zu korrigierenden Körperteil angelegt ist, wobei für die Herstellung eine Fertigungsformung (25) des orthopädischen Stützelement (1) vorgegeben wird, welche von einer Therapieformung (26) des orthopädischen Stützelementes (1) beim Anlegen an das zu korrigierenden Körperteils abweicht, wobei in der Fertigungsformung (25) in dem Überlappungsabschnitt (2) ein Abstand (24) zwischen einem ersten offenen Ende (22) und dem zweiten offenen Ende (23) des orthopädischen Stützelementes (1) existiert, welcher in der Therapierformung (26) entfällt, wobei der Abstand (24) zwischen den beiden offenen Enden (22,23) durch eine geringfügige Biegung des orthopädische Stützelement (1) in der Therapieformung (26) gegenüber der Fertigungsformung (25) entfällt aufweisend die folgenden Schritte:
a) Bereitstellen einer Innenform (3), welche eine Haltung des Körperteils vorgibt,
b) Bereitstellen einer Außenform (4), wobei die Außenform (4) zusammen mit der Innenform (3) eine Gussform (5) zum Herstellen des orthopädischen Stützelementes (1) aus einem Gußwerkstoff (6) bildet,
c) Gießen des Orthopädischen Stützelements (1) mit Hilfe des aus Außenform (4) und Innenform (3) gebildete Gussform (5) und mindestens eines Gußwerkstoffes (6),
wobei die Außenform (4) den Überlappungsabschnitt (2) zumindest abschnittsweise beidseitig umgibt.

2. Verfahren nachAnspruch 1, wobei eine Außenform (4) der Gußform (5) im Bereich des Überlappungsabschnitts (2) eine Oberflächenform (7) aufweist, die zumindest arealweise der Oberflächenform (7) der Innenform (3) entspricht.

3. Verfahren nach einem der Ansprüche 1 oder 2, wobei eine Außenform (4) der Gußform (5) einen Übergangsbereich (8) zu dem Überlappungsabschnitt (2) bildet in welchem die Außenform (4) flächig an der Innenform (3) anliegt.

4. Verfahren nach einem der Ansprüche 1 bis 3, wobei eine Außenform (4) der Gußform (5) mehrteilig ausgeführt ist und ein erster Teil (9) der Außenform (4) eine erste Seite (11) des Überlappungsabschnittes (6) vorgibt während ein zweiter Teil (10) der Außenform (4) eine der ersten Seite (11) gegenüberliegende zweite Seite (12) des Überlappungsabschnittes (6) vorgibt.

5. Verfahren nach einem der Ansprüche 1 bis 4, wobei zumindest eine Außenform (4) der Gußform (5) mit einem dreidimensionalen Druckverfahren hergestellt wird.

6. Verfahren nach einem der vorhergehenden Ansprüche, wobei der Gußwerkstoff (6) ein Silikonwerkstoff ist.

## Claims

1. A method for producing an orthopedic support element (1), comprising an overlapping portion (2) in which the orthopedic support element overlaps itself when it is placed against a body part to be corrected, wherein, for the production, a manufacturing shaping (25) of the orthopedic support element (1) is predefined, which manufacturing shaping differs from a therapy shaping (26) of the orthopedic support element (1) when being placed against the body part to be corrected, wherein, in the manufacturing shaping (25), there is a distance (24) in the overlapping portion (2) between a first open end (22) and the second open end (23) of the orthopedic support element (1) that is not present in the therapy shaping (26), whereby the distance (24) between the two open ends (22, 23) is eliminated by a slight curving of the orthopedic support element (1) in the therapy shaping (26) compared to the manufacturing shaping (25) comprising the following steps:
a) providing an inner mold (3) which specifies the posture of the body part,
b) providing an outer mold (4), wherein the outer mold (4), together with the inner mold (3), forms a casting mold (5) for producing the orthopedic support element (1) from a casting material (6),
c) casting the orthopedic support element (1) using the casting mold (5) formed from the outer mold (4) and the inner mold (3) and at least one casting material (6),
wherein the outer mold (4) surrounds the overlapping section (2) on both sides, at least in certain portions.

2. The method according to claim 1, wherein, in the region of the overlapping portion (2), an outer mold (4) of the casting mold (5) has a surface shape (7) which corresponds to the surface shape (7) of the inner mold (3) at least in certain areas.

3. A method according to one of claims 1 or 2, wherein an outer mold (4) of the casting mold (5) forms a transitional region (8) to the overlapping portion (2), in which transitional region the outer mold (4) rests against the inner mold (3) in a sheet-like manner.

4. A method according to any one of claims 1 to 3, wherein an outer mold (4) of the casting mold (5) has multiple parts, and a first part (9) of the outer mold (4) predefines a first side (11) of the overlapping portion (6) while a second part (10) of the outer mold (4) predefines a second side (12), opposite the first side (11), of the overlapping portion (6).

5. A method according to any one of claims 1 to 4, wherein at least one outer mold (4) of the casting mold (5) is produced by a three-dimensional printing method.

6. A method according to any one of the preceding claims, wherein the casting material (6) is a silicone material.

## Revendications

1. Procédé de fabrication d'un élément de support orthopédique (1), comprenant une section de chevauchement (2), dans laquelle l'élément de support orthopédique se chevauche lui-même lorsqu'il est appliqué sur une partie du corps à corriger, une forme de fabrication (25) de l'élément de support orthopédique (1), qui diffère d'une forme thérapeutique (26) de l'élément de support orthopédique (1) lorsqu'il est appliqué sur la partie du corps à corriger, étant prédéfinie pour la fabrication, un écartement (24) entre une première extrémité ouverte (22) et la deuxième extrémité ouverte (23) de l'élément de support orthopédique (1) existant, dans la forme de fabrication (25), dans la section de chevauchement (2), lequel disparaît dans la forme thérapeutique (26), l'écartement (24) entre les deux extrémités ouvertes (22, 23) disparaissant par une légère flexion de l'élément de support orthopédique (1) dans la forme thérapeutique (26) par rapport à la forme de fabrication (25), comprenant les étapes suivantes :
a) la mise à disposition d'une forme intérieure (3), qui définit une posture de la partie du corps,
b) la mise à disposition d'une forme extérieure (4), la forme extérieure (4) formant, conjointement avec la forme intérieure (3), un moule de coulée (5) destiné à fabriquer l'élément de support orthopédique (1) à partir d'un matériau de coulée (6),
c) le coulage de l'élément de support orthopédique (1) à l'aide du moule de coulée (5) formé par la forme extérieure (4) et la forme intérieure (3) et d'au moins un matériau de coulée (6),
la forme extérieure (4) entourant la section de chevauchement (2) des deux côtés, au moins par sections.

2. Procédé selon la revendication 1, dans lequel une forme extérieure (4) du moule de coulée (5) présente, dans la zone de la section de chevauchement (2), une forme de surface (7) qui correspond, au moins sur certaines zones, à la forme de surface (7) de la forme intérieure (3).

3. Procédé selon l'une des revendications 1 ou 2, dans lequel une forme extérieure (4) du moule de coulée (5) forme une zone de transition (8) vers la section de chevauchement (2), dans laquelle la forme extérieure (4) est en appui surfacique contre la forme intérieure (3).

4. Procédé selon l'une des revendications 1 à 3, dans lequel une forme extérieure (4) du moule de coulée (5) est réalisée en plusieurs parties et une première partie (9) de la forme extérieure (4) définit une première face (11) de la section de chevauchement (6), tandis qu'une deuxième partie (10) de la forme extérieure (4) définit une deuxième face (12) de la section de chevauchement (6), opposée à la première face (11).

5. Procédé selon l'une des revendications 1 à 4, dans lequel au moins une forme extérieure (4) du moule de coulée (5) est fabriquée au moyen d'un procédé d'impression tridimensionnelle.

6. Procédé selon l'une des revendications précédentes, dans lequel le matériau de coulée (6) est un matériau en silicone.
